(19) Europäisches Patentamt
European Patent Office
Office européen des brevets

(11) Publication number : **0 398 033 B1**

(12) # EUROPEAN PATENT SPECIFICATION

(45) Date of publication of patent specification : **25.08.93 Bulletin 93/34**

(51) Int. Cl.$^5$ : **A61K 31/435,** A61K 9/20, A61K 9/22

(21) Application number : **90107387.4**

(22) Date of filing : **19.04.90**

(54) **Oral preparation.**

(30) Priority : **20.04.89 JP 101403/89**

(43) Date of publication of application : **22.11.90 Bulletin 90/47**

(45) Publication of the grant of the patent : **25.08.93 Bulletin 93/34**

(84) Designated Contracting States : **CH DE FR GB IT LI**

(56) References cited :
**EP-A- 0 137 198**
**EP-A- 0 168 044**
**EP-A- 0 301 465**

(73) Proprietor : **FUJISAWA PHARMACEUTICAL CO., LTD.**
**4-7, Doshomachi 3-chome Chuo-ku**
**Osaka-shi Osaka 541 (JP)**

(72) Inventor : **Hata, Takehisa**
**2-4-2, Kayougaoka**
**Nagakakyo-shi, Kyoto 617 (JP)**
Inventor : **Yoshida, Hiromitsu**
**5 Burggravenlaan**
**NL-2313 HM Leiden (NL)**
Inventor : **Yamamoto, Takao**
**1-5-17-403, Wakayamadai, Shimamoto-cho**
**Mishima-gun, Osaka 618 (JP)**
Inventor : **Saito, Takashi**
**2-10-33-101 Kawaramiya**
**Amagasaki-shi, Hyogo 661 (JP)**
Inventor : **Yamaguchi, Hisami**
**5-19-19 Shinkofudai, Toyono-cho**
**Toyono-gun, Osaka 563-01 (JP)**
Inventor : **Imai, Keiji**
**2-3-1, Hirarigaoka**
**Takarazuka-shi, Hyogo 665 (JP)**

(74) Representative : **Türk, Gille, Hrabal, Leifert**
**Brucknerstrasse 20**
**D-40593 Düsseldorf (DE)**

EP 0 398 033 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a pharmaceutical preparation for oral administration of N-(1H-tetrazol-5-yl)-1-phenoxy-4H-quinolizin-4-one-3-carboxamide or a salt thereof (hereinafter referred to briefly as quinolizinone compound) which is of value as an antiallergic agent or an antiulcer agent. More particularly, the invention relates to a controlled-release preparation for improved gastrointestinal absorption comprising said quinolizinone compound and a water-soluble high molecular compound, which can be used with advantage in the field of medicine.

The term 'quinolizinone compound' as it is used in the context of this specification means a compound of the following chemical structure :

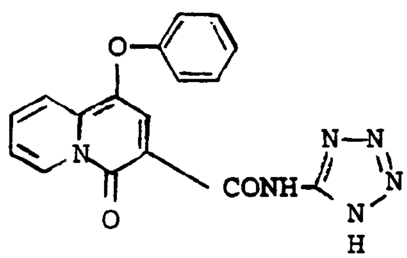

or a salt thereof.

However, since this quinolizinone compound is only sparingly soluble in water, it is poorly absorbed into blood on oral administration in the usual dosage forms, thus being low in bioavailability. Therefore, a new pharmaceutical preparation overcoming this disadvantage has been in demand.

The intensive research undertaken by the inventors of this invention revealed that the above problem could be obviated by formulating said quinolizinone compound with a water-soluble high molecular compound and further that the release of the active compound from such preparation could be controlled as desired by adjusting the proportion of said water-soluble high molecular compound and, if desired, by adding a wax to said preparation or by coating said preparation. This invention has been conceived and developed on the basis of the above findings.

The oral preparation according to this invention comprises the above-mentioned quinolizinone compound and a water-soluble high molecular compound. The coexistence of the quinolizinone compound and the water-soluble high molecular compound eliminates the disadvantage of poor water-solubility of the quinolizinone compound to thereby assure a high bioavailability thereof even when the same compound is administered by the oral route.

In accordance with this invention, a pharmaceutical sustained-release oral preparation can be provided by adjusting the proportion of said water-soluble high molecular compound and, if desired, by adding a wax to the preparation or by coating the preparation.

The quinolizinone compound as the active ingredient of the oral preparation of this invention may be in the hydrate form, and the salt thereof may for example be an alkali metal salt such as the sodium or potassium salt. The most advantageous form of quinolizinone compound is the monohydrate of the quinolizinone sodium salt.

The preferred examples of the water-soluble high molecular compound to be included in the oral preparation of this invention include those used commonly in the related art, such as cellulose derivatives (e.g. hydroxypropylmethylcellulose, hydroxypropylcellulose, methylcellulose, carboxymethylcellulose, sodium carboxymethylcellulose, hydroxyethylcellulose, nitrocellulose and microcrystalline cellulose,), synthetic water-soluble polymers (e.g. polyvinylpyrrolidone, cross-linked polyvinylpyrrolidone and polyethylene oxide,) and polysaccharides (e.g. dextrin). Amongst these compounds, cellulose derivatives are preferred, and hydroxypropylmethylcellulose is the most desirable of all.

The oral preparation of this invention may, if necessary, contain those additives which are routinely employed in pharmaceutical procedures, including disintegrating agents, lubricants, excipients, colors and solubilizers. There is no limitation on dosage form. Thus, the pharmaceutical preparation obtained as above can be provided in such various dosage forms as powders, fine granules, granules, capsules and tablets.

Preferred examples of said disintegrating agents include starches (e.g. potato starch, corn starch, hydroxypropyl-starch, sodium carboxymethyl-starch), cellulose derivatives (e.g. calcium carboxymethylcellulose sodium carboxymethylcellulose, carboxymethylcellulose, low-substitution hydroxypropylcellulose, microcrystalline cellulose) and sodium croscarmellose. Preferred examples of said lubricants include talc, waxes (e.g.

bleached beeswax, hydrogenated oil), stearic acid compounds (e.g. stearic acid, magnesium stearate, calcium stearate). Preferred examples of said excipients include, sugars (e.g. lactose, sucrose, D-mannitol), starches (e.g. corn starch), inorganic calcium salts (e.g. calcium hydrogen phosphate, calcium sulfate). Preferred examples of said colors include tar colors or yellowish rouge. Preferred examples of said solubilizers include magnesium hydroxide, calcium carbonate, sodium bicarbonate and arginine. These are, however, merely illustrative and other additives which are commonly used in the field can be incorporated with success.

The pharmaceutical preparation of this invention can be manufactured by adding said water-soluble high molecular compound and, if necessary, said additives to said quinolizinone compound and, then, processing the resulting mixture into a suitable oral dosage form.

The method for manufacture of the pharmaceutical preparation of this invention is described in detail below.

Thus, the quinolizinone compound is first mixed with the water-soluble high molecular compound and, if necessary, with other known additives. This mixing can be effected by the routine procedure. For further comminution of the particles, the mixture may be crushed, also by the known procedure. The resulting powdery mixture containing the quinolizinone compound and water-soluble high molecular compound can be processed into an optional oral dosage form by known procedures such as pulverization, sieving, kneading, granulation, compression molding and coating.

For the purpose of providing a sustained-release oral preparation of this invention, the above-mentioned mixture is preferably first supplemented with a wax and then, processed into the desired dosage form, or firstly compressed, and then processed into film-coated tablets.

The wax to be used for this purpose may be any wax that is either insoluble or sparingly soluble in water and as such includes vegetable and animal waxes (e.g. carnauba wax or bees-wax), various hydrogenated oils (e.g. hydrogenated soybean oil or hydrogenated castor oil), paraffins (e.g. paraffin wax or microcrystalline wax). These waxes may be used in combination.

The film-coating materials used here should be independent of pH of an outer medium, and may include one such as acrylate resin [e.g. dimethylaminoethylmethacrylate-methylmetacrylic acid copolymer (e.g. Eudragit® E 30 D)], shellac, cellulose derivatives [e.g. ethylcellulose, hydroxypropyl-methylcellulose].

The film-coating materials may, if necessary, contain those additives which are routinely employed in pharmaceutical procedures such as colors (e.g. titanium dioxide or yellowish rouge), plasticizer (e.g. polyethylene glycol), polishing agents (e.g. carnauba wax).

The amount of the coating material is 0.1 to 20, preferably 0.7 to 15, and most preferably 2 to 6, weight percent of the powdery mixture containing the drug, but the amount is not restricted to the above range and should be selected depending on the intended duration of drug release.

The coating treatment may be carried out with a fluid bed granulator in a conventional manner.

However, this is not an exclusive choice but any other known method such as coacervation, interfacial polymerization or spray-drying can be utilized.

The above process yields a pharmaceutical preparation comprising said quinolizinone compound and water-soluble high molecular compound according to this invention. The type and amount of water-soluble high molecular compound and those of each other additive can be selected according to the amount of quinolizinone compound and the desired dissolution or release pattern and duration of action of the quinolizinone compound.

The ratio of quinolizinone compound to water-soluble high molecular compound is selected according to the designed release time of the active compound. While the release time is prolonged as the proportion of water-soluble high molecular compound is increased, the ratio by weight is generally about 1:0.1 to 1:10 preferably 1:0.5 to 1:5, and most preferably 1:1.

When the preparation is coated, the ratio of quinolizinone compound to the coating material is generally about 1:0.01 to 1:2 by weight and preferably about 1:0.05 to 1:0.1 by weight.

When a wax is incorporated in the preparation, the ratio of quinolizinone compound to wax is generally about 1:0.1 to 1:5 by weight and preferably about 1:1 to 1:5 by weight.

The following production and working examples are further illustrative of this invention.

Production Example 1

Tetrahydrate of sodium N-(1H-tetrazol-5-yl)-1-phenoxy-4H-quinolizin-4-one-3-carboxamide (7.08 kg) was added to a mixture of isopropyl alcohol (34 ℓ) and water (22.7 ℓ) and dissolved under warming. To this solution was added charcoal powder and the mixture was stirred on reflux at 79-80°C for 0.5 hour. The charcoal powder was filtered off when hot and the charcoal powder was washed with 60% aqueous isopropyl alcohol. The filtrate and washings were pooled, cooled and then ripened under stirring for 1 hour. The resulting precipitate was recovered by centrifuging, washed with isopropyl alcohol and dried. The above procedure provided monohydrate of sodium N-(1H-tetrazol-5-yl)-1-phenoxy-4H-quinolizin-4-one-3-carboxamide (hereinafter referred to briefly as quinolizinone A) with a water content of 4.9% (5.62 kg).

Example 1

Quinolizinone A (1.0 g) was pulverized with a jet atomizer and this powder was mixed with TC-5R (a trademark of Shin-Etsu Chemical; hydroxypropylmethylcellulose) (1.0 g), L-HPC (a trademark of Shin-Etsu Chemical; low-substitution hydroxypropylcellulose) (1.0 g) and D-mannitol (2.0 g). The resulting mixture was comminuted in a mortar to give a final mixture. This powdery mixture was filled into No.5 capsules to give capsules for oral administration. The composition per capsule was as follows.

| | |
|---|---|
| Quinolizinone A | 10.0 mg |
| TC-5R | 10.0 mg |
| L-HPC | 10.0 mg |
| D-Mannitol | 20.0 mg |
| | 50.0 mg |

Example 2

Quinolizinone A (1.0 g) was pulverized with a jet atomizer and the resulting powder was mixed with TC-5R (1.0 g), L-HPC (1.0 g) and D-mannitol (2.0 g). The mixture was kneaded with distilled water (1.7 ml) and granulated. The granules were dried in vacuo, comminuted in a mortar and sieved to minus 32 mesh to give a mixed powder. This mixed powder was kneaded with carnauba wax (3.0 g) which had been molten at about 80°C and after cooling, the solidified mass was crushed and sieved to minus 20 mesh. To this powder was added magnesium stearate (0.04 g) and the mixture was compression-molded using a 6 mm (dia.) punch in the routine manner to give tablets. The composition per tablet was as follows.

| | |
|---|---|
| Quinolizinone A | 10.0 mg |
| TC-5R | 10.0 mg |
| L-HPC | 10.0 mg |
| D-Mannitol | 20.0 mg |
| Carnauba wax | 30.0 mg |
| Magnesium stearate | 0.4 mg |
| | 80.4 mg |

Example 3

The mixed powder prepared in the same manner as Example 2 was kneaded with molten carnauba wax (about 80°C) (2.0 g) and paraffin (1.0 g). After cooling, the resulting solid mixture was crushed and sieved to minus 20 mesh. To this powder was added magnesium stearate (0.04 g) and the mixture was compression-molded using a 6 mm (dia.) punch to give tablets. The composition per tablet was as follows.

| | |
|---|---|
| Quinolizinone A | 10.0 mg |
| TC-5R | 10.0 mg |
| L-HPC | 10.0 mg |
| D-Mannitol | 20.0 mg |
| Carnauba wax | 20.0 mg |
| Paraffin | 10.0 mg |
| Magnesium stearate | 0.4 mg |
| | 80.4 mg |

Example 4

The mixed powder obtained using TC-5R (3.0 g) in otherwise the same manner as Example 2 was mixed with magnesium stearate (0.04 g) and the resulting mixture was compression-molded using a 6 mm (dia.) punch in the routine manner to give tablets. The composition per tablet was as follows.

| | |
|---|---|
| Quinolizinone A | 10.0 mg |
| TC-5R | 30.0 mg |
| L-HPC | 10.0 mg |
| D-Mannitol | 20.0 mg |
| Magnesium stearate | 0.4 mg |
| | 70.4 mg |

Example 5

Using carnauba wax (2.0 g) and paraffin (1.0 g), the procedure of Example 3 was repeated to give tablets. The composition per tablet was as follows.

| | |
|---|---|
| Quinolizinone A | 10.0 mg |
| TC-5R | 30.0 mg |
| L-HPC | 10.0 mg |
| D-Mannitol | 20.0 mg |
| Carnauba wax | 20.0 mg |
| Paraffin | 10.0 mg |
| Magnesium stearate | 0.5 mg |
| | 100.5 mg |

Example 6

The mixed powder obtained using Quinolizinone A pulverized with a jet atomizer, TC-5R, magnesium hydroxide, Avicel (a trademark of Asahi Chemical Industry; microcrystalline cellulose) and D-mannitol in the same manner as Example 2 was mixed with Ac-Di-Sol (a trademark of Asahi Chemical Industry; sodium croscarmellose) and magnesium stearate, and the resulting mixture was compression-molded using 6 or 8 mm (dia.) punch in the routine manner to give tablets. To the tablets were film-coated with a solution or dispersion of TC-5R, titanium dioxide, yellowish rouge and PEG-6000 (a trademark of Sanyo Chemical Industries; Macrogol-6000) in distilled water, and were polished with Carnauba wax to give film-coated tablets.

The composition per film-coated tablet was as follows.

| | | |
|---|---|---|
| (1) | Quinolizinone A | 5.0 mg |
| | TC-5R | 5.0 mg |
| | Magnesium hydroxide | 1.25 mg |
| | Avicel | 27.0 mg |
| | D-Mannitol | 43.75 mg |
| | Ac-Di-Sol | 7.0 mg |
| | Magnesium stearate | 1.0 mg |
| | subtotal | 90.0 mg |

(a part of film-coating)

| | |
|---|---|
| TC-5R | 2.4 mg |
| Titanium dioxide | 1.12 mg |
| Yellowish rouge | 0.08 mg |
| PEG-6000 | 0.4 mg |
| Carnauba wax | trace amounts |
| subtotal | 4.0 mg |
| total | 94.0 mg |

| | | |
|---|---|---|
| (2) | Quinolizinone A | 20.0 mg |
| | TC-5R | 20.0 mg |
| | magnesium hydroxide | 5.0 mg |
| | Avicel | 54.0 mg |
| | D-Mannitol | 59.0 mg |
| | Ac-Di-Sol | 20.0 mg |
| | Magnesium stearate | 2.0 mg |
| | subtotal | 180.0 mg |

(a part of film-coating)

| | |
|---|---|
| TC-5R | 4.2 mg |
| Titanium dioxide | 1.96 mg |
| Yellowish rouge | 0.14 mg |
| PEG-6000 | 0.7 mg |
| Carnauba wax | trace amounts |
| subtotal | 7.0 mg |
| total | 187.0 mg |

In the pharmaceutical preparation of this invention, the solubility of the quinolizinone compound has been markedly improved over that of the bulk substance so that sufficient bioavailability is assured after oral administration. Furthermore, the invention provides for sustained drug action.

To assist in the understanding of the above-mentioned effects, the data generated by the in vitro dissolution test and oral administration test in dogs of some representative preparations according to this invention are set forth hereunder.

Dissolution Test 1

[I] The preparations used in the test

Preparation A : The capsule according to Example 1

Control preparation : No.5 capsule containing 10 mg of quinolizinone A bulk

[II] Test method

The dissolution rate was determined at timed intervals in accordance with Japanese Pharmacopoeia XI, Dissolution Test, Method 2 (paddle method).

The test conditions were as follows.

Testing apparatus : Manufactured by Toyama Sangyo Co., Ltd.

Solvent and amount : Fluid 2 (pH 6.8), 900 ml

RPM of paddle : 100 rpm.

Determination : Ultraviolet light, 240 nm

[III] Results

The dissolution rates (%) were as follows.

| Test preparation | Time (min.) | |
|---|---|---|
| | 15 | 30 |
| Preparation A | 69.4 | 70.8 |
| Control preparation | 4.7 (10 min.) | 7.9 |

Dissolution Test 2

[I] The preparations used in the test

Preparation B : The tablet according to Example 2

Control preparation : The same bulk as used in Dissolution Test 1

[II] Test method

This test was performed by the same method as Dissolution Test 1.

[III] Results

| Test preparation | Time (min.) | | | |
|---|---|---|---|---|
| | 30 | 60 | 120 | 240 |
| Preparation B | 7.3 | 12.9 | 39.7 | 53.3 |
| Control preparation | 7.9 | 10.7 | 13.6 | - |

Dissolution Test 3

[I] The preparations used in the test

Preparation D : The tablet according to Example 4

Preparation E : The tablet according to Example 5

Control preparation : The same bulk as used in Dissolution Test 1

[II] Test method

This test was performed by the same method as Dissolution Test 1.

[III] Results

| Test preparation | Time (min.) | | | |
|---|---|---|---|---|
| | 30 | 60 | 120 | 240 |
| Preparation D | 20.6 | 38.3 | 62.4 | 69.2 |
| Preparation E | 10.0 | 14.8 | 23.8 | 40.4 |
| Control preparation | 7.9 | 10.7 | 13.6 | - |

Dissolution Test 4

[I] The preparations used in the test

Preparation F : The tablet according to Example 6-(1)

Preparation G : The tablet according to Example 6-(2)

Control preparation : The same bulk as used in Dissolution Test 1

[II] Test method

This test was performed by the same method as Dissolution Test 1.

[III] Results

| Test preparation | Time (min.) | |
|---|---|---|
| | 15 | 30 |
| Preparation F | 96.2 | 97.4 |
| Preparation G | 89.6 | 89.8 |
| Control preparation | 4.7 (10 min.) | 7.9 |

It is apparent from the above test results that the preparations according to this invention, which contain a water-soluble high molecular compound, have been markedly improved in the dissolution pattern of the quinolizinone compound as compared with the bulk substance. That is to say, the disadvantage, i.e. poor solubility, of the quinolizinone compound is remarkably improved in the pharmaceutical preparation of this invention.

Detailed analysis of the foregoing dissolution test results revealed that preparation B obtained by kneading a mixed powder of quinolizinone compound and water-soluble high molecular compound with a wax is retarded in the rate of release compared with preparation A which is free of wax, thus affording a sustained-release feature. Furthermore, the results of dissolution tests 2 and 3 indicated that the use of water-soluble high molecular compound in an increased proportion relative to quinolizinone compound assures not only a better sustained-release feature but also a greater improvement in the solubility of quinolizinone compound.

Still furthermore, the results of dissolution test 4 indicated that preparations F and G obtained by firstly compressing a mixed powder of quinolizinone compound and water-soluble high molecular compound, and then processing into film-coated tablet assures a greater improvement in the solubility of quinolizinone compound.

In view of the common belief that mere admixture of an insoluble drug substance with a water-soluble high molecular compound provides little improvement in the solubility of the drug substance, it is a surprising discovery that such simple admixing resulted in a marked improvement in solubility in the case of this invention.

Oral Administration Test

[I] The preparation used in the test

The same Preparations A and E and Control Preparation as used in the dissolution tests were employed.

[II] Test method

Using 5 male beagle dogs (body weights 8.5-10.5 kg) fasted overnight, the test was carried out by the three-way crossover method.

The dosage was 10 mg as quinolizinone A per animal and each preparation was orally administered. After dosing, blood was sampled from the antebrachial vein and quinolizinone A in the blood sample was promptly assayed by high-performance liquid chromatography (HPLC).

[III] Results

The plasma concentration, maximum plasma concentration (Cmax) and the area under the plasma concentration - time curve ($AUC_{0-24}$) at various time points following oral administration are shown below in the tables. Each value is the mean ± standard error.

| Test preparation | Time (hr.) 0.5 | 1 | 2 | 4 | 8 | 12 | 24 | Cmax (μg/ml) | AUC 0-24 (μg.hr/ml) |
|---|---|---|---|---|---|---|---|---|---|
| Preparation A | 0.49 ±0.12 | 0.93 ±0.14 | 0.94 ±0.17 | 0.61 ±0.03 | 0.43 ±0.08 | - | 0.05 ±0.03 | 1.07 ±0.12 | 7.92 ±0.72 |
| Preparation E | 0.06 ±0.02 | 0.08 ±0.03 | 0.38 ±0.30 | 0.59 ±0.16 | 0.69 ±0.13 | 0.63 ±0.14 | 0.03 ±0.01 | 0.91 ±0.04 | 10.17 ±1.17 |
| Control Preparation | 0.05 ±0.04 | 0.19 ±0.17 | 0.23 ±0.18 | 0.23 ±0.10 | 0.12 ±0.02 | n.d. | n.d. | 0.31 ±0.14 | 1.76 ±0.74 |

n .d.: not detected.

It is apparent from the above absorption data that compared with the quinolizinone A bulk substance, the composition according to this invention has been markedly improved in absorbability into the blood.

Thus, the foregoing results of various experiments indicate clearly that the pharmaceutical preparation of this invention has been remarkably improved in the disadvantage of the quinolizinone compound, that is to say the low solubility thereof in water, and, hence, in the low rate of absorption thereof into blood after oral administration. It is also apparent that the pattern of release and duration of action of the quinolizinone compound can be freely controlled by selecting the types and amounts of water-soluble high molecular compound and other additives. As such, the pharmaceutical preparation of this invention comprising the quinolizinone compound and water-soluble high molecular compound as essential ingredients is of great clinical use.

## Claims

1. A sustained-release pharmaceutical preparation for oral administration which comprises N-(1H-tetrazol-5-yl)-1-phenoxy-4H-quinolizin -4-one-3-carboxamide or a salt thereof as an active ingredient and a water-soluble high molecular compound.

2. The pharmaceutical preparation of claim 1 wherein said water-soluble high molecular compound is a cellulose derivative.

3. The pharmaceutical preparation of claim 2 wherein said cellulose derivative is hydroxypropylmethylcellulose.

4. The pharmaceutical preparation of claim 1, 2 or 3 wherein the active ingredient is monohydrate of sodium N-(1H-tetrazol-5-yl)-1-phenoxy-4H-quinolizin-4-one-3-carboxamide.

5. The pharmaceutical preparation of claim 1, 2, 3 or 4 wherein the weight ratio of the active ingredient to the water-soluble high molecular compound is 1:0.1 to 1:10.

6. The pharmaceutical preparation of claim 1, 2, 3 or 4 wherein the weight ratio of the active ingredient to the water-soluble high molecular compound is 1:0.5 to 1:5.

7. A pharmaceutical preparation of Claim 1, 2, 3, 4, 5 or 6 which is prepared by mixing the active ingredient and the water-soluble high molecular compound with one or more additives.

8. A pharmaceutical preparation of claim 1, 2, 3, 4, 5 or 6 which is prepared by mixing the active ingredient and the water-soluble high molecular compound with one or more additives and compressing the resulting mixed powder, and then processing into film-coated tablets.

## Patentansprüche

1. Pharmazeutisches Präparat mit Langzeit-Wirkung für die orale Verabreichung, das enthält N-(1H-Tetrazol-5-yl)-1-phenoxy-4H-chinolizin-4-on-3-carboxamid oder ein Salz davon als aktiven Bestandteil (Wirkstoff) und eine wasserlösliche hochmolekulare Verbindung.

2. Pharmazeutisches Präparat nach Anspruch 1, worin die wasserlösliche hochmolekulare Verbindung ein Cellulosederivat ist.

3. Pharmazeutisches Präparat nach Anspruch 2, worin das Cellulosederivat Hydroxypropylmethylcellulose ist.

4. Pharmazeutisches Präparat nach Anspruch 1, 2 oder 3, worin der aktive Bestandteil (Wirkstoff) das Monohydrat von Natrium-N-(1H-tetrazol-5-yl)-1-phenoxy-4H-chinolizin-4-on-3-carboxamid ist.

5. Pharmazeutisches Präparat nach Anspruch 1, 2, 3 oder 4, worin das Gewichtsverhältnis zwischen dem aktiven Bestandteil (Wirkstoff) und der wasserlöslichen hochmolekularen Verbindung 1:0,1 bis 1:10 betrrägt.

6. Pharmazeutisches Präparat nach Anspruch 1, 2, 3 oder 4, worin das Gewichtsverhältnis zwischen dem aktiven Bestandteil (Wirkstoff) und der wasserlöslichen hochmolekularen Verbindung 1:0,5 bis 1:5 beträgt.

7. Pharmazeutisches Präparat nach Anspruch 1, 2, 3, 4, 5 oder 6, das durch Mischen des aktiven Bestandteils (Wirkstoffes) und der wasserlöslichen hochmolekularen Verbindung mit einem oder mehr Additiven hergestellt worden ist.

8. Pharmazeutisches Präparat nach Anspruch 1, 2, 3, 4, 5 oder 6, das durch Mischen des aktiven Bestandteils (Wirkstoffes) und der wasserlöslichen hochmolekularen Verbindung mit einem oder mehr Additiven und Pressen des resultierenden Pulvergemisches und anschließendes Verarbeiten zu mit einem Film überzogenen Tabletten hergestellt worden ist.

## Revendications

1. Préparation pharmaceutique à libération retardée pour l'administration orale qui comprend du N-(1H-tétrazol-5-yl)-1-phénoxy-4H-quinolizin-4-one-3-carboxamide ou un de ses sels comme ingrédient actif et un composé de masse moléculaire élevée soluble dans l'eau.

2. Préparation pharmaceutique selon la revendication 1, dans laquelle ledit composé de masse moléculaire élevée soluble dans l'eau est un dérivé de la cellulose.

3. Préparation pharmaceutique selon la revendication 2, dans laquelle ledit dérivé de la cellulose est l'hydroxypropylméthylcellulose.

4. Préparation pharmaceutique selon la revendication 1, 2, ou 3, dans laquelle l'ingrédient actif est le monohydrate de N-(1H-tétrazol-5-yl)-1-phénoxy-4H-quinolizin-4-one-3-carboxamide de sodium.

5. Préparation pharmaceutique selon la revendication 1, 2, 3 ou 4, dans laquelle le rapport pondéral de l'ingrédient actif au composé de masse moléculaire élevée soluble dans l'eau est 1:0,1 à 1:10.

6. Préparation pharmaceutique selon la revendication 1, 2, 3 ou 4, dans laquelle le rapport pondéral de l'ingrédient actif au composé de masse moléculaire élevée soluble dans l'eau est 1:0,5 à 1:5.

7. Préparation pharmaceutique selon la revendication 1, 2, 3, 4, 5, ou 6, qui est préparée en mélangeant l'ingrédient actif et le composé de masse moléculaire élevé soluble dans l'eau avec un ou plusieurs additifs.

8. Préparation pharmaceutique selon la revendication 1, 2, 3, 4, 5 ou 6, qui est préparée en mélangeant l'ingrédient actif et le composé de masse moléculaire élevée soluble dans l'eau avec un ou plusieurs additifs

et en comprimant la poudre mélangée obtenue, puis en faisant un traitement pour obtenir des comprimés revêtus d'une pellicule.